**Europäisches Patentamt**

**European Patent Office**

**Office européen des brevets**

(19)

(11) Veröffentlichungsnummer: **0 207 250 B1**

(12) **EUROPÄISCHE PATENTSCHRIFT**

(45) Veröffentlichungstag der Patentschrift:
04.04.90

(51) Int. Cl.⁴: **A61M 31/00**

(21) Anmeldenummer: 86105792.5

(22) Anmeldetag: 26.04.86

(54) **Medizinische Depotsonde.**

(30) Priorität: 02.07.85 CH 2826/85

(43) Veröffentlichungstag der Anmeldung:
07.01.87 Patentblatt 87/2

(45) Bekanntmachung des Hinweises auf die Patenterteilung:
04.04.90 Patentblatt 90/14

(84) Benannte Vertragsstaaten:
AT BE DE FR GB IT NL SE

(56) Entgegenhaltungen:
WO-A-82/03174
AT-A- 362 048
DE-A- 3 115 763
US-E- 25 788

(73) Patentinhaber: **GEBRÜDER SULZER AKTIENGESELLSCHAFT**, Zürcherstrasse 9,
CH-8401 Winterthur(CH)

(72) Erfinder: **Billeter, Werner**, Brühlbergstrasse 25,
CH-8400 Winterthur(CH)
Erfinder: **Bittmann, Peter, Dr.**, Langackerstrasse 126,
CH-8704 Herrliberg(CH)

(74) Vertreter: **Sparing Röhl Henseler Patentanwälte European Patent Attorneys**, Rethelstrasse 123,
D-4000 Düsseldorf 1(DE)

**Beschreibung**

Die Erfindung betrifft eine medizinische Depotsonde, bestehend aus einem Sondenkopf und einem Sondenendteil, bei der mindestens der Sondenkopf ein Hohlkörper ist und der Sondenkopf aus einzelnen, aneinandergereihten Segmenten gebildet ist, die mit Medikamententrägern gefüllt sind, wobei die Segmente mit Durchtrittsöffnungen für den Durchtritt von Flüssigkeit versehen sind, und wobei ferner zwischen zwei in axialer Richtung aufeinander folgenden Medikamententrägern jeweils die Flexibilität erhöhende Gelenkzonen geschaffen sind.

Eine Depotsonde der genannten Art ist bekannt aus der DE-OS 3 115 763; diese Sonde, die für eine zeitlich begrenzte Implantation in den menschlichen Körper bestimmt ist, hat die Aufgabe, eine dosierte Medikamentenabgabe über einen gewissen Zeitraum unmittelbar und lokal, beispielsweise in einen Infektionsherd hinein, zu gewährleisten. Sie besteht aus einem hohlen Sondenkopf, der mit dem Wirkstoff beladene Medikamententräger enthält, und aus einem Sondenendteil; sie ist aus einem nicht porösen glatten Kunststoff gefertigt. Der Sondenkopf ist dabei in einzelne, mit dem Medikamententräger gefüllte Segmente unterteilt, die eine Vielzahl auf dem Sondenkopf-Hohlkörper in Umfangs- und in axialer Richtung verteilte Durchtrittsöffnungen für Körperflüssigkeiten in der einen und für Wirkstoff in der anderen Richtung aufweisen. Zwischen den Segmenten sind massive elastische Zwischenstücke vorgesehen, die – wie die Segmente – von einem Kanal in Richtung der Sondenlängsachse und von einem Querkanal durchsetzt sind, die den Abfluß von Sekreten aus dem Infektionsbereich fördern sollen. Die Verbindungen zwischen Segmenten bzw. dem Sondenendteil einerseits und den Zwischenstücken andererseits erfolgt dabei über Klebe-, Schraub-, Steck- oder andere sich nicht ungewollt lösende Verbindungen.

Depotsonden der geschilderten Art sollten eine Reihe von sich zum Teil widersprechenden Eigenschaften haben, um ihre Funktion optimal erfüllen zu können:

So müssen die Medikamententräger eine Mindestfestigkeit haben, damit insbesondere beim Einsatz ins Muskelgewebe oder an Stellen, die starken Beugungen unterworfen sind, kein Abbröckeln erfolgt, bei dem nach dem Entfernen der Sonde Rückstandspartikel im Körper verbleiben können.

Andererseits soll der Sondenkopf eine möglichst große Flexibilität haben, damit die Sonde zum einen nicht zu mechanischen Reizungen führt und zum anderen in anatomisch vorgebildete Kanäle erleichtert eingeschoben werden kann.

Eine weitere, von medizinischer Seite erhobene Forderung besteht darin, daß die Wandstärken des Hohlkörpers besonders im Sondenkopf möglichst gering sein sollen, um möglichst "flache" Durchtrittsöffnungen zu erhalten, in die nur wenig Gewebe hineinwachsen kann; denn beim Entfernen der Sonde wird dieses Gewebe auf- oder zerrissen, was zu vermeidbaren Schmerzen führt.

Hohe Flexibilität des Sondenkopfes und relativ dünne Wandstärken können beim Abbiegen der Sonde dazu führen, daß die Durchtrittsöffnungen stark verformt werden, so daß sie sich im äußeren Bereich einer Krümmung, wo der Hohlkörper gedehnt wird, stark vergrößern, während sie auf der Innenseite, an der die Hohlkörperwand gestaucht wird, verkleinern oder sogar ganz verschließen.

Es ist daher Aufgabe der Erfindung, eine Depotsonde mit hochflexiblem Sondenkopf zu schaffen, bei der derartige Verzerrungen der Durchtrittsöffnungen möglichst vermieden sind. Diese Aufgabe wird mit der Erfindung dadurch gelöst, daß die Erstreckung eines Medikamententrägers in Richtung der Sondenlängsachse höchstens das 3fache, vorzugsweise höchstens das doppelte, der längsten linearen Abmessung des Sondenquerkopfquerschnittes beträgt. Die beanspruchte Relation zwischen Sondenaußendurchmesser und axialer Länge eines Medikamententrägers bedingt relativ "kurze" Medikamententräger, die hintereinander aufgereiht werden. Die geforderte Flexibilität wird dabei durch die Gelenkzonen zwischen den einzelnen Medikamententrägern erreicht.

Die Gelenkzonen sind mit Vorteil durch Einschnürungen des Hohlkörpers gebildet, wobei die Querschnittsfläche in den Gelenkzonen mit Vorteil zwischen 35 und 70% der ungestörten Querschnittsfläche des Hohlkörpers betragen kann. Darüberhinaus bilden sich durch die Leerräume der Einschnürungen zwischen den Medikamententrägern Hohlräume, in die beim Abbiegen die am Innenradius einer Krümmung gestauchte Sondenwand "ausweichen" kann.

Es ist auch möglich, die Gelenkzonen, z.B. als Faltenbälge, auszubilden; allerdings wird dadurch die glatte Außenfläche, die für ein möglichst schmerzfreies Extrahieren der Sonde vorteilhaft ist, gestört.

Um möglichst geringe Wandstärken im Sondenkopf zu ermöglichen, ist es zweckmäßig, wenn man jedem Medikamententräger in Richtung der Sondenlängsachse eine einzige Durchtrittsöffnung zuordnet; die Durchtrittsöffnungen sind zudem im unverzerrten Zustand vorzugsweise kreisförmig, um einen hohen "Ausreißwiderstand" bei Zugbelastungen zu erreichen.

Die Größe der Sonde ist in erster Linie durch die anatomischen Gegebenheiten bestimmt. Viele lokal zu behandelnden Infektionen treten an den Körperextremitäten auf; auch in diesen Fällen ist es von großer Bedeutung, daß die Sonde das postoperativ anfallende Sekret aus dem Infektionsbereich abzuleiten vermag. Aus rein konstruktiven Gründen ist es jedoch bei kleinen Sondendurchmessern – 5 mm und kleiner – nicht möglich, den bei der bekannten Sonde vorgesehenen zentralen Längskanal unterzubringen, ohne eine untragbare Einbuße an Wirkstoffbeladung des Sondenkopfes in Kauf nehmen zu müssen. Soll die Depotsonde daher gleichzeitig als klassischer Drainageschlauch dienen, so ist es zweckmäßig, wenn Sondenendteil und Sondenkopf ein einziger durchgehender Hohlkörper aus einem flexiblen Werkstoff sind, wobei mindestens der sondenkopfnahe Bereich des Sondenendteils mit Drainageöffnungen versehen ist. Mit einem derartigen Sondenendteil wird gleichzeitig erreicht, daß freigesetzter Wirkstoff immer mit zu behandelndem Gewebe unmittelbar in Berührung kommt, ehe er besonders bei einem Anschluß der Drainage an eine Absaugung mit dem Sekretstrom ausgeschwemmt wird. Um beim Anlegen einer Absaugung einen Zusammenbruch der Drainage zu verhindern, ist es möglich, die Wandstärke des Hohlkörpers im Sondenendteil gegenüber derjenigen des Sondenkopfes zu vergrößern, die allein durch die erforderliche Zugfestigkeit bestimmt ist; ein Zusammenfallen des hohlen Querschnittes kann jedoch auch durch im Sondenendteil angeordnete Stege verhindert werden. Weiterhin kann es zweckmäßig sein, zwischen dem Sondenkopf und dem Sondenendteil eine Trennwand vorzusehen, um eine direkte Absaugung von im abzusaugenden Sekret gelöstem Wirkstoff zu verhindern.

Die Zugfestigkeit besonders des Sondenkopfes läßt sich verbessern, wenn in Umfangsrichtung pro Medikamententräger nur zwei einander diametral gegenüberliegende Durchtrittsöffnungen vorgesehen sind, die bei zwei axial aufeinander folgenden Medikamententrägern jeweils um 90° gegeneinander versetzt sind; selbstverständlich kann eine derartige Anordnung auch für die Drainageöffnungen des Sondenendteils getroffen sein.

Im folgenden wird die Erfindung anhand von Ausführungsbeispielen im Zusammenhang mit der Zeichnung näher erläutert.

Fig. 1 ist ein Längsschnitt durch eine erste Ausführungsform, die einen kreisförmigen Querschnitt hat;
Fig. 2 ist der Schnitt II–II von Fig. 1;
Fig. 3 und 4 stellen – ebenfalls im Längs- und im Querschnitt – eine zweite Ausführungsform mit einem rechteckigen Querschnitt dar.

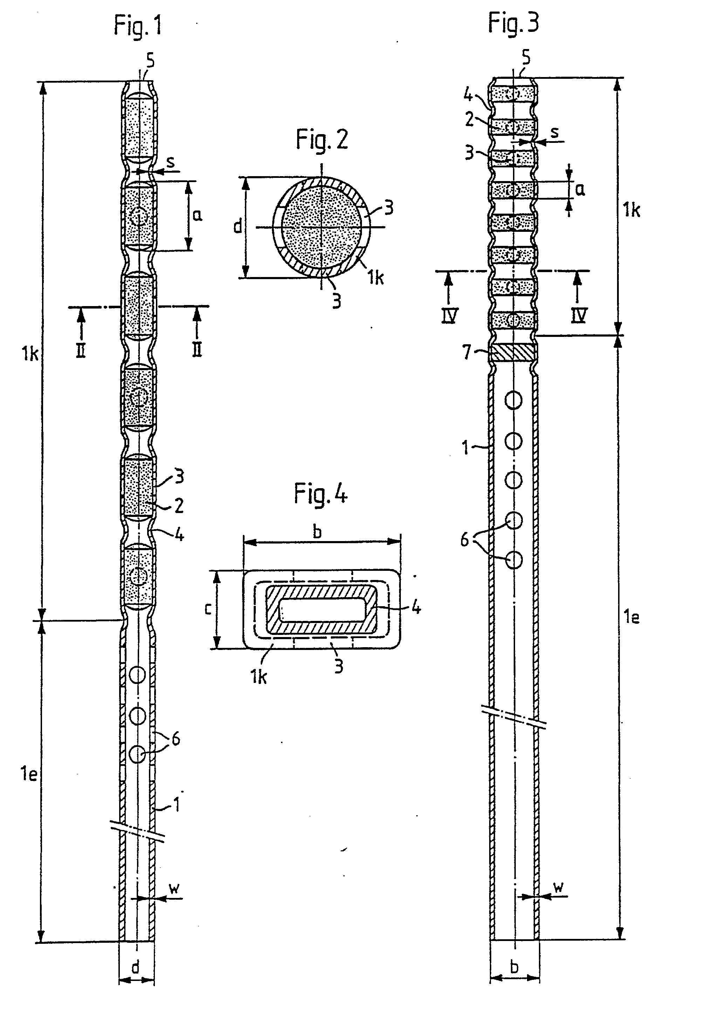

Bei beiden gezeigten Ausführungsbeispielen, die sich nur durch ihre unterschiedliche Querschnittsformen unterscheiden, besteht die Depotsonde aus einem einzigen durchgehenden Hohlkörper 1 (Fig. 1), der einen Sondenkopf 1k und einen Sondenendteil 1e bildet. Die Wandstärke w im Sondenendteil 1e ist dabei größer als diejenige s im Sondenkopf 1k. Ihre Absolutwerte richten sich nach der "Größe" der Sonde, d.h. in erster Linie nach deren Durchmesser. Diese Größe ist von den medizinischen Einsatzgebieten abhängig; beispielsweise sind Sonden mit größeren Außendurchmessern d für den Einsatz im Rumpf vorgesehen, während Sonden für den Einsatz in den Extremitäten geringere Durchmesser aufweisen. Im Finger- und Zehenbereich setzt man mit Vorteil relativ flache rechteckige Sondenformen ein.

Der Hohlkörper 1 der Sonde ist beispielsweise hergestellt aus einem weichgemachten PVC-Schlauch medizinischer Qualität, der im Handel erhältlich ist. Dieser Schlauch wird für seine Formung zur erfindungsgemäßen Depotsonde einer thermischen Verarbeitung unterworfen, wie sie für derartige Kunststoffe bekannt ist.

Der Sondenkopf 1k enthält in Richtung der Sondenlängsachse hintereinander aufgereiht Medikamententräger 2, die aus wachsartigen Substanzen bestehen, die in Wasser absolut unlöslich sind und Schmelzpunkte zwischen 50 und 100°C haben. Als Beispiele dafür seien genannt Fettalkohole – wie Hexadecanol, Octadecanol, Eicosanol – oder Esterwachse auf der Basis von Montanwachsen wie z.B. "Höchst-Wachs E Pharma" der Firma Höchst. Die Medikamententräger sind mit einem Wirkstoff beladen, der aus einem wasserlöslichen Antibiotikum oder einem wasserlöslichen Desinfektionsmittel besteht, die beide für eine Lokalapplikation geeignet sein müssen. Für die erste Wirkstoff-Klasse seien Aminoglykoside (Gentamicin, Tobramicin, Amikacin), Polypeptid Antibiotika (Tyrothricin, Bacitracin) oder Nitrofurane erwähnt, für die zweite PVP-Jod und andere jodophore Polymere und Biguanidine.

Erfindungsgemäß betragen die Längen a der Medikamententräger 2 in Richtung der Sondenlängsachse höchstens das 2- oder 3-fache der Außendurchmesser d des Hohlköprers 1.

Im Bereich jedes Medikamententrägers 2 sind in der Wand des Sondenkopfes 1k zwei einander diametral gegenüberliegende Durchtrittsöffnungen 3 vorhanden, die beispielsweise aus der Schlauchwand herausgestanzt sind. Diese Durchtrittsöffnungen 3 sind – sofern es die absoluten Abmessungen des Hohlkörpers 1 zulassen – bei axial aufeinander folgenden Medikamententrägern 2 um 90° versetzt zueinander (Fig. 1). Dies bringt den Vorteil, daß nicht zwei Mantelbereiche in ihrer Zugfestigkeit axial durchgehend geschwächt sind. Eine weitere Durchtritsöffnung 5 bildet das stirnseitige Ende des Sondenkopfes 1k. Die Öffnungsquerschnitte der Durchtrittsöffnungen 3 werden so groß wie möglich gewählt; das sie begrenzende Merkmal besteht darin, daß der Hohlkörper 1 eine Zugfestigkeit haben muß, die zum Herausziehen der Sonde nach dem Auslösen der Wirkstoffe ausreicht.

Zwischen den einzelnen Medikamententrägern 2 sind Gelenkzonen 4 vorgesehen, die aus Einschnürungen der Schlauchwand bestehen und – wie erwähnt – durch eine thermische Bearbeitung des Schlauches mit Hilfe einer Kernform hergestellt werden. In ihren linearen Dimensionen – Durchmesser d bzw. Seitenlängen b und c – sind die Einschnürungen auf etwa 60 bis 80% ihrer ungestörten Abmessungen verkleinert, so daß sich Verringerungen der Querschnittsflächen auf etwa 35 bis 70% des ungestörten Querschnitts ergeben.

Im kopfnahen Bereich des Sondenendteils 1e, der an die innerste Einschnürung 4 anschließt, sind – wenn möglich ebenfalls alternierend um 90° versetzt zueinander (Fig. 1) – Drainageöffnungen 6 eingestanzt.

Ein Abfließen von Sekret kann gefördert werden, wenn der Sondenendteil 1e mit seinem freien Ende an eine nicht dargestellte Absauganlage angeschlossen ist. In diesem Fall kann zwischen Sondenkopf 1k

und Sondenendteil 1e eine Trennwand 7 (Fig. 3) vorgesehen sein, um ein direktes Absaugen von Wirkstoff aus dem innersten Medikamententräger 2 zu verhindern. Im einfachsten Fall kann diese Trennwand 7 aus einem nicht mit Wirkstoff beladenen Medikamententräger 2 bestehen.

Weiterhin kann man auf die Länge des Sondenendteils 1e dessen Hohlform stützende Stege vorsehen, die nicht ausdrücklich gezeigt sind. Sie haben die Aufgabe, beim Anlegen des Vakuums einer Absaugung ein Kollabieren des Sondenendteils 1e zu verhindern.

Abschliessend seien tabellenartig Daten für je ein Beispiel der gezeigten Ausführungsformen angegeben:

|  | Fig. 1 und 2 | Fig. 3 und 4 |
|---|---|---|
| Einsatzgebiet | Körperrumpf | Finger und Zehen |
| **Sonde** | | |
| Querschnitt | kreisförmig | rechteckig |
| Gesamtlänge mm | 400 | 250 |
| Länge des Sondenkopfes mm | 60 | 45 |
| Aussenabmessungen mm | 6 | 4 x 2 |
| Wandstärke des Sondenkopfes mm | 0,5 | 0,3 |
| Wandstärke des Sondenendteils mm | 0,75 | 0,6 |
| **Durchtritts- und Drainageöffnungen** | | |
| Form | kreisförmig | kreisförmig |
| Durchmesser mm | 2 | 1,5 |
| **Medikamententräger** | | |
| Anzahl | 6 | 8 |
| Form | zylindrisch | quaderförmig |
| axiale Länge mm | 6,5 | 3 |
| Durchmesser bzw. Dicke mm | 5 | 1,4 |
| Material | Fettalkohol | |
| Wirkstoff | Aminoglykosid | Jod-Präparat |

**Patentansprüche**

1. Medizinische Depotsonde, bestehend aus einem Sondenkopf (1k) und einem Sondenendteil (1e), bei der mindestens der Sondenkopf (1k) ein Hohlkörper ist, und der Sondenkopf (1k) aus einzelnen, aneinandergereihten Segmenten gebildet ist, die mit Medikamententrägern (2) gefüllt sind, wobei die Segmente mit Durchtrittsöffnungen (3) für den Durchtritt von Flüssigkeit versehen sind, und wobei ferner zwischen zwei in axialer Richtung aufeinander folgenden Medikamententrägern (2) jeweils die Flexibilität erhöhende Gelenkzonen (4) geschaffen sind, dadurch gekennzeichnet, daß die Erstreckung (a) eines Medikamententrägers (2) in Richtung der Sondenlängsachse höchstens das 3-fache, vorzugsweise höchstens das doppelte, der längsten linearen Abmessungen (d, b) des Sondenkopfquerschnittes beträgt.

EP 0 207 250 B1

2. Depotsonde nach Anspruch 1, dadurch gekennzeichnet, daß die Gelenkzonen (4) durch Einschnürungen des Hohlkörpers (1) gebildet sind.

3. Depotsonde nach Anspruch 2, dadurch gekennzeichnet, daß die Querschnittsfläche in den Gelenkzonen (4) 35 bis 70% der ungestörten Querschnittsfläche des Hohlkörpers (1) beträgt.

4. Depotsonde nach Anspruch 1, dadurch gekennzeichnet, daß jedem Medikamententräger (2) in Richtung der Sondenlängsachse eine einzige Durchtrittsöffnung (3) zugeordnet ist.

5. Depotsonde nach Anspruch 1, dadurch gekennzeichnet, daß Sondenendteil (1e) und Sondenkopf (1k) ein einziger durchgehender Hohlkörper (1) aus einem flexiblen Werkstoff sind.

6. Depotsonde nach Anspruch 5, dadurch gekennzeichnet, daß in Umfangsrichtung pro Medikamententräger (2) zwei einander diametral gegenüberliegende Durchtrittsöffnungen (3) vorgesehen sind, die bei zwei axial aufeinander folgenden Medikamententrägern (2) jeweils um 90° gegeneinander versetzt sind.

7. Depotsonde nach Anspruch 5, dadurch gekennzeichnet, daß die Wandstärke (w) des Hohlkörpers (1) im Sondenendteil (1e) gegenüber derjenigen (s) des Sondenkopfes (1k) vergrößert ist.

8. Depotsonde nach Anspruch 5, dadurch gekennzeichnet, daß mindestens der sondenkopfnahe Bereich des Sondenendteils (1e) mit Drainageöffnungen (6) versehen ist.

9. Depotsonde nach Anspruch 5, dadurch gekennzeichnet, daß der Hohlkörper (1) im Sondenendteil (1e) ein Zusammenfallen des hohlen Querschnittes verhindernde Stege aufweist.

10. Depotsonde nach Anspruch 8, dadurch gekennzeichnet, daß zwischen dem Sondenkopf (1k) und dem Sondenendteil (1e) eine Trennwand (7) vorgesehen ist.

**Claims**

1. A medical depot probe comprising a probe head (1k) and a probe end part (1b), at least the head (1k) being a hollow member and the head (1k) being made up of a row of individual adjacent segments filled with drug carriers (2), the segments being formed with openings (3) for fluid to flow through, and flexibility-increasing articulated regions (4) being formed between each pair of successive drug carriers (2) in the axial direction, characterised in that the extension (a) of a drug carrier (2) in the direction of the longitudinal axis of the probe is not greater than three times, preferably not greater than twice, the longest linear dimension (d, b) of the probe-head cross-section.

2. A depot probe according to claim 1, characterised in that the articulated regions (4) are formed by constrictions in the hollow member (1).

3. A depot probe according to claim 2, characterised in that the cross-sectional area in the articulated regions (4) is 35 to 70% of the undisturbed cross-sectional area of the hollow member (1).

4. A depot probe according to claim 1, characterised in that each drug carrier (2) is associated with a single through opening (3) in the direction of the longitudinal axis of the probe.

5. A depot probe according to claim 1, characterised in that the end part (1e) and the head (1k) of the probe constitute a single continuous hollow member (1) made of flexible material.

6. A depot probe according to claim 5, characterised in that two diametrically opposite through openings (3) are provided in the peripheral direction for each drug carrier (2) and are offset by 90° from one another in the case of each pair of axially successive drug carriers (2).

7. A depot probe according to claim 5, characterised in that the wall thickness (w) of the hollow member (1) in the end part (1e) of the probe is greater than the wall thickness (s) of the probe head (1k).

8. A depot probe according to claim 5, characterised in that at least the region of the end part (1e) near the probe head is formed with drainage openings 6.

9. A depot probe according to claim 5, characterised in that the hollow members (1) has webs in the end part (1e) which prevent the hollow cross-section from collapsing.

10. A depot probe according to claim 8, characterised in that a partition (7) is provided between the head (1k) and the end part (1e) of the probe.

**Revendications**

1. Sonde de libération retardée d'un médicament, constituée d'une tête de sonde (1k) et d'une partie terminale de sonde (1e), dans laquelle au moins la tête de sonde (1k) est un corps creux, et la tête de sonde (1k) est formée par différents segments juxtaposés qui sont remplis de supports de médicaments (2), les segments étant pourvus d'orifices de passage (3) pour le passage de liquide, et des zones d'articulation (4), augmentant la flexibilité, étant créées en outre entre deux supports de médicaments (2) successifs, en direction axiale, caractérisée en ce que l'extension (a) d'un support de médicament (2) dans la direction de l'axe longitudinal de la sonde, est au plus égale à trois fois, de préférence au plus à deux fois, la dimension linéaire la plus longue (d, b) de la section transversale de la tête de sonde.

2. Sonde de libération retardée d'un médicament selon la revendication 1, caractérisée en ce que les zones d'articulation (4) sont formées par des étranglements du corps creux (1).

3. Sonde de libération retardée d'un médicament selon la revendication 2, caractérisée en ce que la superficie de la section transversale dans les zones d'articulation (4) varie entre 35 et 70% de la superficie de la section transversale libre du corps creux (1).

5

4. Sonde de libération retardée d'un médicament selon la revendication 1, caractérisée en ce qu'un seul orifice de passage (3) est associé à chaque support de médicament (2) dans la direction de l'axe longitudinal de la sonde.

5. Sonde de libération retardée d'un médicament selon la revendication 1, caractérisée en ce que la partie terminale de la sonde (1e) et la tête de sonde (1k) constituent un seul corps creux (1) continu réalisé dans un matériau souple.

6. Sonde de libération retardée d'un médicament selon la revendication 5, caractérisée en ce qu'il est prévu, dans la direction périphérique, deux orifices de passage (3) diamétralement opposés, par support de médicament (2), qui sont décalés de 90° l'un par rapport à l'autre, pour deux supports de médicaments (2) successifs axialement.

7. Sonde de libération retardée d'un médicament selon la revendication 5, caractérisée en ce que l'épaisseur de paroi (w) du corps creux (1) est supérieure dans la partie terminale (1e) de la sonde à celle (s) de la tête de sonde (1k).

8. Sonde de libération retardée d'un médicament selon la revendication 5, caractérisée en ce que la zone de la partie terminale de la sonde (1e), voisine de la tête de sonde, est pourvue d'orifices de drainage (6).

9. Sonde de libération retardée d'un médicament selon la revendication 5, caractérisée en ce que le corps creux (1) comporte, dans la partie terminale de sonde (1e), des entretoises qui empêchent un effondrement de la section transversale creuse.

10. Sonde de libération retardée d'un médicament selon la revendication 8, caractérisée en ce qu'une cloison (7) est prévue entre la tête de sonde (1k) et la partie terminale de sonde (1e).

Fig. 1

Fig. 2

Fig. 3

Fig. 4